# EUROPEAN PATENT SPECIFICATION

(11) **EP 0 985 658 B1**
(45) Date of publication and mention of the grant of the patent: **12.05.2004**
(21) Application number: 99909211.7
(22) Date of filing: 17.03.1999
(51) Int. Cl.: C07C 229/08, C07C 227/18, C07C 309/30, C07C 303/32

(54) **PROCESS FOR PRODUCING L-VALINE BENZYL ESTER P-TOLUENESULFONATE**
VERFAHREN ZUR HERSTELLUNG VON L-VALINBENZYLESTER-P-TOLUOLSUFONATEN
PROCEDE DE PRODUCTION DE L-VALINE BENZYLE ESTER P-TOLUENESULFONATE

(30) Priority: 17.03.1998 JP 6672598
(43) Date of publication of application: 15.03.2000
(73) Proprietor: SUMIKA FINE CHEMICALS Co., Ltd., Osaka-shi, Osaka 555-0021 (JP)
(72) Inventor: TAGAMI, Yayoi, Sumika Fine Ch..Co.,Ltd., Osaka-shi, Osaka 555-0021 (JP); KATSURA, Tadashi, Sumika Fine Chemicals Co., Ltd., Osaka-shi, Osaka 555-0 (JP); ITAYA, Nobushige, Sumika Fine Chemicals Co., Ltd., Osaka-shi, Osaka 555-0 (JP)
(74) Representative: Marchant, James Ian
(86) International application number: PCT/JP1999/001334
(87) International publication number: WO 1999/047488

(56) References cited:
- JP-A- 5 230 022
- JP-A- 7 206 792
- US-A- 4 199 499
- US-A- 5 362 912
- ZERVAS L ET AL: "STUDIES ON ARGININE PEPTIDES. I. INTERMEDIATES IN THE SYNTHESIS OF N-TERMINAL AND C-TERMINAL ARGININE PEPTIDES" JOURNAL OF ORGANIC CHEMISTRY, AMERICAN CHEMICAL SOCIETY. EASTON, US, November 1957 (1957-11), pages 1515-1521, XP001074255 ISSN: 0022-3263
- LASH T D ET AL: "SYNTHESIS OF PYRROLES FROM BENZYL ISOCYANOACETATE" SYNTHESIS, GEORG THIEME VERLAG. STUTTGART, DE, no. 2, 1994, pages 170-172, XP002919135 ISSN: 0039-7881
- LASH TIMOTHY D. et al., "Synthesis of Pyrroles from Benzyl Isocyanoacetate", SYNTHESIS, 1994, No. 2, p. 170-172, XP002919135

## Description

### TECHNICAL FIELD

The present invention relates to a process for preparing L-valine benzyl ester p-toluenesulfonate, and more specifically to a process for preparing L-valine benzyl ester p-toluenesulfonate useful, for example, as an intermediate for pharmaceuticals such as antihypertensives.

### BACKGROUND ART

Conventionally, L-valine benzyl ester p-toluenesulfonate has been prepared by reacting L-valine with benzyl alcohol in the presence of p-toluenesulfonic acid in benzene under azeotropic dehydration conditions, thereafter cooling the resulting reaction solution, diluting the solution with benzene and diethyl ether, and crystallizing the L-valine benzyl ester p-toluenesulfonate (J. Org. Chem., vol.22, pp.1515-1521, 1957).

However, since this process employs benzene which has been thought to exhibit carcinogenicity as a solvent, and also uses a large amount of ether showing high inflammability together with benzene for the dilution of the reaction solution, the process has a defect in safety. Therefore, the process is industrially disadvantageous.

US Patent No. 5,362,912 discloses a method of preparation of the p-toluenesulfonic acid salt of a benzyl ester of valine whereby L-valine, benzyl alcohol and p-toluenesulfonic acid monohydrate are stirred in toluene, heated to reflux and subsequently cooled to room temperature and poured into ethyl ether, whereupon said salt is precipitated.

Lash *et al*. describe the preparation of benzyl glycinate p-toluenesulfonate, its subsequent conversion to benzyl N-formylglycinate, and subsequent dehydration to benzyl isocyanoacetate.

US Patent No. 4,199,499 discloses compounds of the formula: 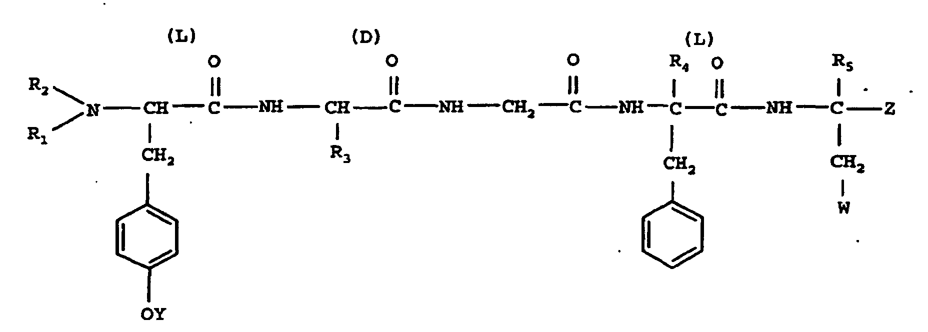 and pharmaceutically acceptable non-toxic acid addition salts thereof, including those prepared from p-toluenesulfonic acid.

Consequently, there has been desired the development of a process for safely, conveniently and industrially advantageously preparing L-valine benzyl ester p-toluenesulfonate.

An object of the present invention is to provide a process for safely, conveniently and industrially advantageously preparing L-valine benzyl ester p-toluenesulfonate.

### DISCLOSURE OF THE INVENTION

According to the present invention, there is provided a process for preparing L-valine benzyl ester p-toluenesulfonate comprising reacting L-valine with benzyl alcohol in the presence of p-toluenesulfonic acid in toluene under azeotropic dehydration conditions at a temperature of 100° to 150°C; cooling the reaction solution to a temperature of 60° to 90°C; inoculating a seed crystal of the L-valine benzyl ester p-toluenesulfonate and cooling the reaction solution at a rate of 3° to 15°C/h to 0° to 70°C, to crystallise the L-valine benzyl ester p-toluenesulfonate from the reaction solution.

In the present invention, use of toluene as the solvent has an advantage of being more excellent in safety to human bodies compared with benzene since the solvent does not contain benzene which would impart carcinogenicity to the human bodies. Toluene is industrially widely used for various purposes and allows easy crystallization of the L-valine benzyl ester p-toluenesulfonate prepared.

It is desirable that the amount of toluene used is not less than 250 parts by weight and preferably 500 parts by weight from the viewpoint of easy collection of crystals by filtration, and that the amount of toluene used is not more than 3,000 parts by weight and preferably not more than 2,000 parts by weight from the viewpoint of economics, on the basis of 100 parts by weight of L-valine. When the amount of toluene used is not less than 500 parts by weight per 100 parts by weight of L-valine, there is provided an advantage that the L-valine benzyl ester p-toluenesulfonate can be crystallized by cooling the reaction solution after the completion of the reaction. Also, when toluene is used in an amount of less than 500 parts by weight per 100 parts by weight of L-valine, the L-valine benzyl ester p-toluenesulfonate can be efficiently crystallized by adding the toluene to the resulting reaction solution in an amount within the range that the total amount of toluene does not exceed 3,000 parts by weight per 100 parts by weight of L-valine after the completion of the reaction.

An aliphatic hydrocarbon solvent having 5 to 8 carbon atoms such as heptane or hexane can be added to the toluene from the viewpoint of improvement of yield. In this case, it is preferable that the amount of the aliphatic hydrocarbon solvent is 200 to 500 parts by weight per 100 parts by weight of L-valine from the viewpoints of improvement of yield and operational properties for collecting the solvent.

It is desirable that the amount of benzyl alcohol used is not less than one mol and preferably not less than 1.5 mol per one mol of L-valine in order to proceed the reaction at a sufficient rate, and it is also desirable that the amount of benzyl alcohol is not more than 6 mol and preferably not more than 5 mol from the viewpoints of yield and economics.

The p-toluenesulfonic acid can be in the form of anhydride or hydrate. It is desirable that the amount of p-toluenesulfonic acid used is not less than one mol and preferably not less than 1.1 mol per one mol of L-valine in order to proceed the reaction at a sufficient rate, and that the amount of p-toluenesulfonic acid is not more than 1.5 mol and preferably not more than 1.2 mol from the viewpoint of economics.

The reaction can be carried out by mixing L-valine, benzyl alcohol, p-toluenesulfonic acid and toluene, and thereafter heating the resulting mixed solution under azeotropic dehydration conditions to 100° to 150°C or so.

The completion of the reaction of L-valine with benzyl alcohol can be confirmed, for instance, by no generation of azeotropic water, or by the attainment of the production ratio to saturation on high performance liquid chromatography.

After the completion of the reaction, the reaction solution obtained is cooled to 0° to 70°C and preferably 0° to 10°C or so, and thereby the resulting L-valine benzyl ester p-toluenesulfonate can be crystallized. When the reaction solution is cooled, the cooling is carried out to crystallize by adjusting the cooling rate to 3° to 15°C/h. This prevents the crystals from adhering to a reaction vessel and shortens the operating time.

When the L-valine benzyl ester p-toluenesulfonate is crystallized, the reaction solution is seeded with a seed crystal, and thereafter the resulting solution is cooled from the viewpoints of prevention of rapid crystallization and generation of uniformly crystallized crystals.

In the present invention, the above-mentioned aromatic solvent, toluene, can be added to the reaction solution obtained after the completion of the reaction in order to efficiently crystallize the L-valine benzyl ester p-toluenesulfonate as aforementioned. Monochlorobenzene or xylene may be used as alternatives to toluene at this stage in the reaction. The aromatic solvent can be added to the reaction solution obtained just after the completion of the reaction or the cooled reaction solution.

When the L-valine benzyl ester p-toluenesulfonate obtained is crystallized, a saturated hydrocarbon such as hexane or heptane can be used together with the aromatic solvent in order to decrease the solubility of the L-valine benzyl ester p-toluenesulfonate to easily crystallize them. The amount of the saturated hydrocarbon used is not particularly limited, and it is preferable that the amount of the saturated hydrocarbon is usually 50 to 200 parts by volume or so per 100 parts by volume of the total amount of the aromatic solvent used.

In the present invention, for example, a seed crystal of the L-valine benzyl ester p-toluenesulfonate prepared in advance can be inoculated in the reaction solution after cooling the solution to a temperature of 60° to 90°C in order to easily crystallize the L-valine benzyl ester p-toluenesulfonate from the reaction solution obtained.

The L-valine benzyl ester p-toluenesulfonate crystallized can be isolated by an operation such as filtration.

The L-valine benzyl ester p-toluenesulfonate thus obtained can be suitably used as an intermediate for pharmaceuticals, for example, antihypertensives, and the like.

The present invention will be more specifically described by the following examples, however, the present invention is not restricted only to the examples.

### Example 1

A 1-L four-necked flask equipped with a thermometer, a stirrer and a Dean-Stark apparatus was charged with 19.1 g (163 mmol) of L-valine, 53.1 g (491 mmol) of benzyl alcohol, 37.4 g (197 mmol) of p-toluenesulfonic acid monohydrate and 281 ml of toluene, and the contents were mixed and then heated. Toluene containing water began to distill away at the internal temperature of the reaction solution of around 110°C. The reaction solution was heated to 120°C.

After the reaction was allowed to continue for 8 hours, 133 ml of toluene was added thereto. When the reaction solution was cooled to 75°C, a seed crystal of L-valine benzyl ester p-toluenesulfonate was inoculated in the solution, and the resulting solution was cooled to 10°C at a cooling rate of 6°C/h. The resulting crystals were collected by filtration, washed with 126 ml of toluene at 10°C, and dried to obtain 53.0 g of L-valine benzyl ester p-toluenesulfonate (apparent yield based on L-valine: 85.7%, content 95.0%, net yield 81.4%).

### Example 2

A 500-mL four-necked flask equipped with a thermometer, a stirrer and a Dean-Stark apparatus was charged with 20.0 g (171 mmol) of L-valine, 73.8 g (682 mmol) of benzyl alcohol, 35.7 g (188 mmol) of p-toluenesulfonic acid monohydrate and 57 ml of toluene, and the contents were mixed and then heated. Toluene containing water began to distill away at the internal temperature of the reaction solution of around 110°C. The reaction solution was heated to 123°C.

After the reaction was allowed to continue for 5 hours, 235 ml of toluene was added thereto. When the reaction solution was cooled to 73°C, a seed crystal of L-valine benzyl ester p-toluenesulfonate was inoculated in the solution, and the resulting solution was cooled to 0°C. The resulting crystals were collected by filtration, washed with 150 ml of toluene at 0°C, and dried to obtain 57.1 g of L-valine benzyl ester p-toluenesulfonate (apparent yield based on L-valine: 88.1%, content 82.7%, net yield 72.9%).

### Example 3

A 2-L four-necked flask equipped with a thermometer, a stirrer and a Dean-Stark apparatus was charged with 70.0 g (598 mmol) of L-valine, 258.5 g (2390 mmol) of benzyl alcohol, 125.0 g (657 mmol) of p-toluenesulfonic acid monohydrate and 1 L of toluene, and the contents were mixed and then heated. Toluene containing water began to distill away at the internal temperature of the reaction solution of around 101°C. The reaction solution was heated to 118°C.

After the reaction was allowed to continue for 5 hours, a seed crystal of L-valine benzyl ester p-toluenesulfonate was inoculated in the solution, and the resulting solution was cooled to 0°C. The resulting crystals were collected by filtration, washed with 300 ml of toluene at 0°C, and dried to obtain 186.2 g of L-valine benzyl ester p-toluenesulfonate (apparent yield based on L-valine: 82.1%, content 88.8%, net yield 72.9%).

### Example 4

A 1-L four-necked flask equipped with a thermometer, a stirrer and a Dean-Stark apparatus was charged with 35.0 g (299 mmol) of L-valine, 129.2 g (1195 mmol) of benzyl alcohol, 62.5 g (329 mmol) of p-toluenesulfonic acid monohydrate and 100 ml of toluene, and the contents were mixed and then heated. Toluene containing water began to distill away at the internal temperature of the reaction solution of around 110°C. The reaction solution was heated to 122°C.

After the reaction was allowed to continue for 5 hours, 200 ml of heptane and 300 ml of toluene were added thereto. When the reaction solution was cooled to 85°C, a seed crystal of L-valine benzyl ester p-toluenesulfonate was inoculated in the solution, and the resulting solution was cooled to 5°C. The resulting crystals were collected by filtration, washed with 150 ml of toluene at 5°C, and dried to obtain 100.0 g of L-valine benzyl ester p-toluenesulfonate (apparent yield based on L-valine: 88.2%, content 82.3%, net yield 73.4%).

### Comparative Example 1

A 500-mL four-necked flask equipped with a thermometer, a stirrer and a Dean-Stark apparatus was charged with 20.0 g (171 mmol) of L-valine, 73.8 g (682 mmol) of benzyl alcohol, 35.7 g (188 mmol) of p-toluenesulfonic acid monohydrate and 50 ml of benzene. The contents were mixed and then heated, and subjected to azeotropic dehydration.

After the reaction was allowed to continue for 5 hours, 240 ml of benzene and 400 ml of diethyl ether were added thereto. When the reaction solution was cooled to 70°C, a seed crystal of L-valine benzyl ester p-toluenesulfonate was inoculated in the solution, and the reaction solution was cooled to 8°C. The resulting crystals were collected by filtration, washed with 150 ml of benzene at 10°C, and dried to obtain 50.5 g of L-valine benzyl ester p-toluenesulfonate (apparent yield based on L-valine: 78%, content 94.2%, net yield 73.5%).

From the above results, according to the processes of the examples, it is found that the L-valine benzyl ester p-toluenesulfonate is safely, conveniently and industrially advantageously prepared without using benzene which has been conventionally used.

### INDUSTRIAL APPLICABILITY

According to the process of the present invention, L-valine benzyl ester p-toluenesulfonate can be safely, conveniently and industrially advantageously prepared. The L-valine benzyl ester p-toluenesulfonate obtained can be suitably used as an intermediate for pharmaceuticals such as antihypertensives.

## Claims

1. A process for preparing L-valine benzyl ester p-toluenesulfonate **characterized by** reacting L-valine with benzyl alcohol in the presence of p-toluenesulfonic acid in toluene under azeotropic dehydration conditions at a temperature of 100° to 150°C; cooling the reaction solution to a temperature of 60° to 90°C; inoculating a seed crystal of the L-valine benzyl ester p-toluenesulfonate and cooling the reaction solution at a rate of 3° to 15°C/h to 0° to 70°C, to crystallize the L-valine benzyl ester p-toluenesulfonate from the reaction solution.

2. The process for preparing L-valine benzyl ester p-toluenesulfonate according to claim 1, wherein the amount of toluene used is 250 to 3,000 parts by weight per 100 parts by weight of L-valine.

## Patentansprüche

1. Verfahren zur Herstellung von L-Valin-benzylester-p-toluolsulfonat, **gekennzeichnet durch** Umsetzung von L-Valin mit Benzylalkohol in Anwesenheit von p-Toluolsulfonsäure in Toluol unter azeotropen Entwässerungsbedingungen bei einer Temperatur von 100° bis 150°C; Kühlen der Reaktionslösung auf eine Temperatur von 60° bis 90°C; Inokulieren eines Impfkristalls des L-Valinbenzylester-p-toluolsulfonats und Kühlen der Reaktionslösung mit einer Geschwindigkeit von 3° bis 15 °C/h auf 0° bis 70°C, um das L-Valinbenzylester-p-toluolsulfonat aus der Reaktionslösung zu kristallisieren.

2. Verfahren zur Herstellung von L-Valinbenzylester-p-toluolsulfonat nach Anspruch 1, worin die Menge an verwendetem Toluol 250 bis 3000 Gew.-Teile pro 100 Gew.-Teile L-Valin beträgt.

## Revendications

1. Procédé de préparation de L-valine benzyle ester p-toluènesulfonate **caractérisé par** la réaction de L-valine avec de l'alcool benzylique en présence d'acide p-toluènesulfonique dans du toluène dans des conditions de déshydratation azéotropiques à une température de 100 à 150°C ; refroidissement de la solution réactionnelle à une température de 60° à 90°C ; inoculation d'un germe cristallin du L-valine benzyle ester p-toluènesulfonate et refroidissement de la solution réactionnelle à une vitesse de 3 à 15°C/h de 0° à 70°C, pour cristalliser le L-valine benzyle ester p-toluènesulfonate à partir de la solution réactionnelle.

2. Procédé de préparation de L-valine benzyle ester p-toluènesulfonate selon la revendication 1, dans lequel la quantité de toluène utilisée représente de 250 à 3 000 parties en poids pour 100 parties en poids de L-valine.
